(19) Europäisches Patentamt
European Patent Office
Office européen des brevets

(11) Numéro de publication: **0 167 426 B1**

(12) # FASCICULE DE BREVET EUROPEEN

(45) Date de publication du fascicule du brevet: **31.01.90**

(51) Int. Cl.⁴: **C 07 D 207/34**, A 61 K 31/40

(21) Numéro de dépôt: **85401027.9**

(22) Date de dépôt: **24.05.85**

(54) **Nouvelles compositions pharmaceutiques à action immuno-suppressive.**

(30) Priorité: **25.05.84 FR 8408200**

(43) Date de publication de la demande: **08.01.86 Bulletin 86/2**

(45) Mention de la délivrance du brevet: **31.01.90 Bulletin 90/5**

(84) Etats contractants désignés: **AT BE CH DE FR GB IT LI LU NL SE**

(56) Documents cités: **EP-A- 0 001 989**

(73) Titulaire: **Albert ROLLAND S.A. Société dite, 49, Rue St-André-des-Arts, F-75006 Paris (FR)**

(72) Inventeur: **Bessin, Pierre, 1 Allee Bossuet, F-91380 Chilly-Mazarin (FR)**
Inventeur: **Bonnet, Jacqueline, 19 Rue Charcot, F-75013 Paris (FR)**

(74) Mandataire: **Burtin, Jean-François, Cabinet GEFIB 55 Rue Boissonade, F-75014 Paris (FR)**

Il est rappelé que: Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

EP 0 167 426 B1

## Description

L'invention se rapporte à des dérivés de l'acide N-methyl pyrole carboxylique.

Elle a plus particulièrement pour objet des dérivés aroylés des acides N-alcoyl pyrrole carboxylique.

L'invention a spécifiquement pour objet des compositions pharmaceutiques manifestant des propriétés immuno-suppressives renfermant à titre de principe actif au moins un dérivé (4-aroyl pyrrolyl-2) carboxylique de formule générale I

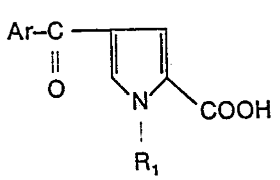

(I)

dans laquelle Ar est choisi dans le groupe constitué par un phényle, un halogeno-phényle, un naphtyl-1 ou un naphtyl-2

$R_1$ est égal à méthyle ou phényle

ou un de ses sels avec une base minérale ou organique en association ou en mélange avec un excipient ou un véhicule inerte non toxique, pharmaceutiquement acceptable.

On connaissait déjà des acides pyrrolyl -2-carboxyliques comme décrits dans le brevet américain 2 479 972. Ces composés ont des propriétés anesthésiques locales.

On connaît également par le brevet américain 3 865 840 des acides 5-aroyl pyrrolyl alcanoïques. Ces composés sont utilisables comme agents anti-inflammatoires.

On connaît encore les dérivés d'acide pyrrole carboxylique de formule générale

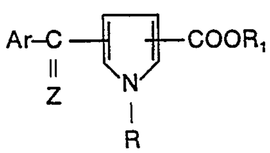

décrits dans le brevet français 2 405 246. Ils manifestent des propriétés uricosuriques, ou tout au moins ils augmentent la vitesse d'élimination du rouge phénol chez le rat comme le font les agents uricosuriques connus.

Or, il a été trouvé maintenant, et c'est sur quoi repose la présente invention, que pour certains composés rentrant dans la formule générale du brevet français 2 405 246 et pour d'autres composés apparentés ne rentrant pas dans le cadre dudit brevet, on a mis en évidence une activité du type immuno-suppressive.

Celle-ci se concrétise par une inhibition du phénomène des rosettes observé par mise en présence de cellules de rate de souris $C_{57}$ $BL_6$ avec des hématies hétérologues de mouton.

Cette inhibition est constatée «in vitro» pour des concentrations en composé de formule générale I allant de 5 à 50 µg/ml. C'est ainsi que l'activité de l'acide N-methyl 4-(naphtoyl-2) pyrrolyl-2 carboxylique est équivalente à celle de l'anti-métabolite pris comme substance de référence: l'Azathioprine ($IC_{50}$ = 6 µg/ml).

Contrairement à l'Azathioprine, les composés de formule générale I sont peu toxiques et surtout dépourvus de toxicité sur les éléments figurés du sang.

Les compositions pharmaceutiques selon l'invention sont utiles pour le traitement des maladies auto-immunes, telles que le lupus erythemateux, la sclérose en plaque, la polyarthrite rhumatoïde et pour la prévention des rejets de greffe.

D'une manière générale, les compositions pharmaceutiques selon l'invention renferment entre 2,5 mg et 1 g de principe actif par prise unitaire, et de préférence entre 5 et 500 mg de principe actif.

Les compositions pharmaceutiques manifestant des propriétés immuno-suppressives, selon l'invention, sont destinées à être administrées par la voie parentérale, la voie orale, la voie rectale, la voie permuqueuse, ou la voie percutanée.

A cette fin, les compositions pharmaceutiques seront présentées sous la forme de comprimés nus ou enrobés, de dragées, de pilules, de capsules, de gélules, de gouttes, de solutions ou suspensions buvables, de solutés ou de suspensions injectables, de suppositoires, de solutions dans un solvant polaire pour l'usage percutané.

La posologie journalière peut varier dans de larges proportions en fonction de la maladie immune qu'il convient de traiter, de l'ancienneté de la maladie et des atteintes organiques que cette maladie a déjà entraînées.

Les composés préférés pour cet usage sont les acides 4-(2-bromobenzoyl) N-methyl pyrrolyl-2 carboxylique, 4-(naphtoyl-1) N-methyl pyrroyl-2 carboxylique et 4-(naphtoyl-2) N-methyl pyrrolyl-2 carboxylique.

Dans ce qui précède, les sels des composés de formule I avec une base minérale ou organique peuvent être définis comme des sels de métaux alcalins, comme le sodium, le potassium, le lithium, l'ammonium ou le rubidium; des sels de métaux alcalino-terreux comme le calcium ou le strontium; le magnésium, l'aluminium ou les métaux ferreux. On peut également y inclure les sels d'alcoylamines, de cyclo-alcoylamines, d'arylalcoyl-amines, de pyridyl-alcoylamines, de furylalcoylamines, d'hydroxy-alcoylamines, d'amino acides, de desoxy-osamines, d'amino cyclitols, de guanidines substituées, de poly peptides ou composés similaires.

Les composés de formule générale I sont obtenus, selon le procédé de brevet français 2 405 246 ou un procédé équivalent. Les exemples suivants illustrent l'invention sans toutefois la limiter.

Exemple I

Comprimés à 25 mg d'acide 4-(naphtoyl-2) N-methyl pyrrolyl-2 carboxylique

| | |
|---|---|
| Acide (Naphtoyl-2) N-méthyl-pyrrolyl-2 carboxylique | 25 g |
| Amidon de blé | 180 g |
| Amidon de maïs | 220 g |
| Silice colloïdale | 10 g |
| Polymère d'éthylène-glycol et de propylène glycol commercialisé sous la marque Pluronid F 68 | 25 g |
| Stéarate de magnésium | 40 g |
| Ethyl cellulose | 20 g |
| Talc | 15 g |

Pour 1000 comprimés terminés à 0 g 560 en moyenne.

Exemple II

Comprimés à 30 mg d'acide N-methyl 4-(Naphtoyl-1) pyrrolyl-2 carboxylique

| | |
|---|---|
| Acide 4-(naphtoyl-1) N-methyl pyrrolyl-2 carboxylique | 30 g |
| Phosphate de magnésium | 150 g |
| Sulfate de calcium | 260 g |
| Gomme arabique | 15 g |
| Talc | 35 g |

Pour 1000 comprimés terminés à 0 g 55 en moyenne

Exemple III

Gélules renfermant 25 mg d'acide 4-(2-bromobenzoyl) N-methyl-pyrrolyl-2 -carboxylique

| | |
|---|---|
| Acide 4-(2-bromobenzoyl) N-methyl pyrrolyl-2-carboxylique | 25 g |
| Lactose | 125 g |
| Mannitol | 10 g |
| Talc | 12 g |
| Stéarate de magnésium | 13 g |

pour 1000 gélules à 0 g 190 en moyenne

Exemple IV

Etude pharmacologique des propriétés immuno-suppressives des composés de formule générale I.

L'activité immuno-suppressive a été mise en particulier en évidence sur des modèles de maladie auto-immune de rat:

- poly-arthrite à l'adjuvant de Freund en traitement curatif ou préventif,
- encéphalite allergique du rat Lewis.

Les compositions selon l'invention sont efficaces sur l'arthrite à l'adjuvant de Freund à partir de la dose journalière de 200 mg/kg par voie orale, administrée à la phase d'état de la maladie (du 21ème au 35ème jour après injection de l'adjuvant: en traitement curatif). Dans les mêmes conditions l'azathioprine est inactive.

Lorsque le traitement quotidien est administré à des fins préventives à partir de la veille de l'injection d'adjuvant de Freund, les compositions selon l'invention sont actives à partir d'une dose de 100 mg/kg de principe actif par jour administré par voie orale. Sur ce test, l'azathioprine est active à partir de 20 mg/kg.

Les compositions selon l'invention protègent les rats de l'encéphalomyélite allergique:

- à la dose de 200 mg/kg de principe actif pendant deux jours: protection de 50%
- à la dose de 400 mg/kg de principe actif: protection de 90% (l'azathioprine protège à 90% à la dose de 100 mg/kg). Les compositions selon l'invention, même administrées à la dose de 400 mg/kg par jour pendant 30 jours, ne modifient pas le nombre des hématies et des leucocytes, alors que l'azathioprine à la dose de 20 mg/kg par jour pendant ce même laps de temps provoque une baisse importante du nombre des éléments figurés du sang.

Les compositions selon l'invention sont également utiles pour le traitement de l'anémie hémolytique acquise auto-immune, de la maladie de Behcet du Pemphigus bulleux et vulgaris, du diabète précoce insulino-dépendant, des endocrinopathies auto-immunes, pour la prévention et le traitement du rejet de transplantation d'organes, pour la prévention et le traitement des réactions de rejet anti-hôte lors de greffes de moelle osseuse.

**Revendications**

L'invention a pour objet:

1. Les compositions pharmaceutiques à propriétés immuno-suppressives renfermant à titre de principe actif au moins un composé de formule générale I

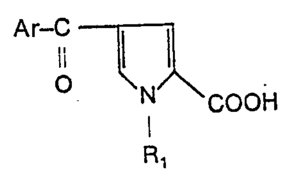

(I)

dans laquelle Ar est choisi dans le groupe constitué par un phényle, un halogeno-phényle, un naphtyl-1 et un naphtyl-2,

et $R_1$ est un méthyle ou un phényle

en association ou en mélange avec un excipient ou un véhicule inerte, non toxique, pharmaceutiquement acceptable.

2. Une composition pharmaceutique à propriétés immuno-suppressives selon la revendication 1 dans laquelle le principe actif est un sel de base minérale ou organique d'un composé de formule générale I.

3. Une composition pharmaceutique à propriétés immuno-suppressives selon l'une des revendications 1 ou 2 dans laquelle la teneur en principe actif s'échelonne de 2,5 à 1000 mg par prise unitaire.

4. Une composition pharmaceutique selon la revendication 1 ou 2 dans laquelle l'excipient ou le véhicule est un de ceux adaptés pour l'administration par voie orale, parentérale.

5. Une composition pharmaceutique selon la revendication 1 ou 2 dans laquelle le principe actif

est l'acide 4-(naphtoyl-1) N-methyl-pyrrolyl-2 carboxylique ou un de ses sels avec une base minérale ou organique.

6. Une composition pharmaceutique selon la revendication 1 ou 2 dans laquelle le principe actif est l'acide 4-(naphtoyl-2) N-methyl-pyrrolyl-2 carboxylique ou un de ses sels avec une base minérale ou organique.

7. Une composition pharmaceutique selon la revendication 1 ou 2 dans laquelle le principe actif est l'acide 4-(2-bromobenzoyl) N-methyl-pyrrolyl-2 carboxylique ou un de ses sels avec une base minérale ou organique.

8. Un procédé d'obtention d'une composition pharmaceutique à propriétés immuno-suppressives selon la revendication 1 qui consiste à associer ou à mélanger un composé de formule générale I

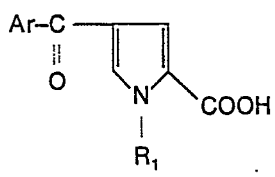

(I)

dans laquelle Ar et $R_1$ ont des définitions fournies précédemment
ou un de ses sels avec une base minérale ou organique
avec un excipient ou un véhicule inerte non toxique pharmaceutiquement acceptable.

## Claims

1. Pharmaceutical compositions endowed with immunosuppressive properties which contain as active ingredient at least one compound of the general formula I

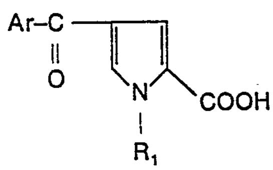

(I)

wherein Ar is a substituent selected from the group consisting of phenyl, halogenophenyl, naphthyl-1 and naphtyl-2
and $R_1$ is methyl or phenyl
admixed or in conjunction with an inert non-toxic pharmaceutically-acceptable carrier or vehicle.

2. A pharmaceutical composition endowed with immunosuppressive properties according to claim 1 wherein the active ingredient is a salt of an organic or inorganic base with a compound of general formula I.

3. A pharmaceutical composition endowed with immunosuppressive properties according to any of claims 1 or 2 in which the amount of active ingredient ranges from 2,5 to 1000 mg unit dosage.

4. A pharmaceutical composition according to claim 1 or 2 in which the carrier or vehicle is one of those suitable for administration per oral or parenteral ways.

5. A pharmaceutical composition according to claim 1 or 2 in which the active ingredient is 4-(Naphtoyl-1) N-methyl pyrrolyl-2 carboxylic acid or a salt thereof with an organic or inorganic base.

6. A pharmaceutical composition according to claims 1 or 2 wherein the active ingredient is 4-(Naphtoyl-2) N-methyl pyrrolyl-2 carboxylic acid or a salt thereof with an organic or inorganic base.

7. A pharmaceutical composition according to claims 1 or 2 wherein the active ingredient is 4-(2-bromobenzoyl) N-methyl pyrrolyl-2 carboxylic acid.

8. A process for producing a pharmaceutical composition endowed with immunosuppressive properties according to claim 1 which consists in mixing or adjuncting a compound of general formula I

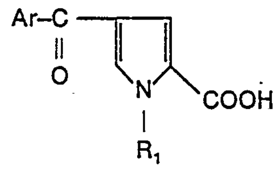

(I)

or a salt thereof with an organic or inorganic base together with an inert non-toxic, pharmaceutically-suitable carrier or vehicle.

## Patentansprüche

1. Pharmazeutische Zusammensetzungen mit immuno-senkenden Eigenschaften, die als aktive Wirkstoff mindenstens eine Verbindung der allgemeinen Formel I

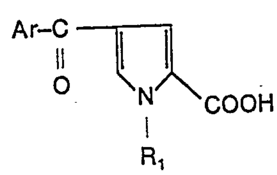

(I)

worin Ar für eine Gruppe die aus Phenyl, Halogensubstituierte phenyl, Naphtyl-1 und Naphtyl-2 gewählt ist, steht
und R methyl oder phenyl ist
enthalten, in Vermischung oder Verbindung mit einem inerten ungiftige pharmazeutisch-verträgliche Verdünnungsmittel oder Vehikel.

2. Eine pharmazeutische Zusammensetzung mit immuno-senkenden Eigenschaften nach Anspruch 1, worin der aktive Wirkstoff ein Salz einer anorganischen oder organischen Base mit einer Verbindung der allgemeinen Formel I ist.

3. Eine pharmazeutische Zusammensetzung mit immuno-senkenden Eigenschaften nach Anspruch 1 oder Anspruch 2 worin der Anteil von aktive Wirkstoff von 2,5 bis 1000 mg bei einzelne Dosis erstreckt.

4. Eine pharmazeutische Zusammensetzung nach Anspruch 1 oder Anspruch 2, worin das Verdünnungsmittel oder Vehikel eines aus diejenigen die für orale oder parenterale Abreichung geeignet sind, ist.

5. Eine pharmazeutische Zusammensetzung nach Anspruch 1 oder Anspruch 2 worin der aktive Wirkstoff 4-(Naphtoyl-1) N-methyl pyrrolyl-2 carbonsäure oder ein Salz davon mit einer anorganischen oder organischen Base, ist.

6. Eine pharmazeutische Zusammensetzung nach Anspruch 1 oder Anspruch 2 worin der aktive Wirkstoff 4-(Naphtoyl-2) N-methyl pyrrolyl-2 carbonsäure oder ein Salz davon mit einer organischen oder anorganischen Base, ist.

7. Eine pharmazeutische Zusammensetzung nach Anspruch 1 oder Anspruch 2 worin der aktive Wirkstoff 4-(2-Bromobenzoyl) N-methyl pyrrolyl-2 carbonsäure ist.

8. Verfahren für die Herstellung der pharmazeutische Zusammensetzungen mit immuno-senkenden Eigenschaften nach Anspruch 1, das aus einer Mischung oder Verbindung eines Produktes der allgemeinen Formel I

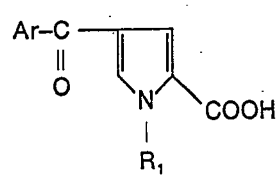

(I)

oder ein Salz davon mit einer anorganischen oder organischen Base zusammen mit einem inerten, ungiftischen, pharmazeutisch-verträglischen Verdünnungsmittel oder Vehikel besteht.